# EUROPÄISCHE PATENTANMELDUNG

(11) **EP 1 095 632 A2**
(43) Veröffentlichungstag der Anmeldung: **02.05.2001**
(21) Anmeldenummer: 00250351.4
(22) Anmeldetag: 26.10.2000
(51) Int. Cl.: A61F 2/06

(54) **Stent mit geschlossener Struktur**

(30) Priorität: 26.10.1999 DE 19951607
(71) Anmelder: BIOTRONIK Mess- und Therapiegeräte GmbH & Co Ingenieurbüro Berlin, 12359 Berlin (DE)
(72) Erfinder: Lootz, Daniel, 18055 Rostock (DE)
(74) Vertreter: Eisenführ, Speiser & Partner

(57) **Zusammenfassung**

Die Erfindung betrifft einen Stent mit einem rohrförmigen Abschnitt, die Ausnehmungen aufweist. Der erfindungsgemäße Stent zeichnet sich dadurch aus, daß die Ausnehmungen im wesentlichen V-förmig ausgebildet sind.

## Beschreibung

Die Erfindung betrifft einen Stent mit einem rohrförmigen Abschnitt, der Ausnehmungen aufweist.

Derartige Stents sind aus dem Stand der Technik in vielfältiger Weise bekannt. Diese Stents werden u.a. im Zusammenhang mit der perkutanen transluminalen Angioplastie (PCTA, Percutaneous Transluminal Balloon Angioplasty) in der Gefäßchirurgie des Herzens verwendet. Stents können jedoch auch dazu dienen, andere Körperöffnungen aufzuweiten oder aufgeweitet zu halten. Diesem medizinischen Verfahren geht zunächst die Bestimmung des Ortes der Verengung eines Herzkranzgefäßes voraus. Anschließend wird ein sogenannter Angioplastieballon in der Arterie, die die Verengung, die sogenannte Stenose aufweist, an den Ort der Stenose verbracht und dort aufgeblasen. Durch die radial nach außen gerichtete Kraft des aufgeblasenen Ballons wird die Verengung aufgeweitet und im optimalen Fall der ursprüngliche Durchtrittsquerschnitt der zuvor verengten Arterie wieder hergestellt. Neben der erfolgreichen Aufweitung des Gefäßes kann es jedoch zu Nebenwirkungen kommen, die lokale Risse in der Arterie, Zersetzungen und Vorsprünge von Plättchen in das Lumen der Arterie umfassen, so daß es trotz der Aufweitung zu einer Blockade des Gefäßes kommen kann. Darüber hinaus ist es möglich, daß durch elastisches Zurückfedern der Gefäßwand und/oder durch das Wachstum der Intima des Gefäßes erneut eine Stenose auftreten kann. Dies tritt statistisch innerhalb von sechs Monaten bei über 30% der Patienten auf, die mit PCTA behandelt wurden.

Um nun unmittelbar nach der Aufweitung des Gefäßes eine relativ glatte Innenwand des Gefäßes sicherzustellen und eine erneute Stenose vermeiden zu können, wurden die eingangs genannten Stents entwickelt. Diese kleinen Röhrchen dienen u.a. im Zusammenhang mit der PCTA dazu, den durch die Ballonangioplastie erzeugten Gefäßdurchtrittsquerschnitt aufrecht zu erhalten, um somit einen langfristigen Erfolg der PCTA sicherzustellen.

Der Erfolg dieses sogenannten Stenting hängt u.a. auch davon ab, wie gleichförmig sich der Stent an die Gefäßwand anlegen kann. Denn umso gleichförmiger die Gefäßwand abgestützt wird, um so wahrscheinlicher ist es, daß es im Bereich der Stents nicht erneut zu Gefäßverengungen kommt. Dabei bewirkt eine gleichmäßige Stent-Struktur eine relativ glatte Gefäßinnenoberfläche und bei einer relativ glatten Gefäßinnenoberfläche können sich Blutpartikel nur schwer an diese anlagern. Darüber hinaus werden auch Wucherungen der Intima in das Gefäßinnere stärker durch eine gleichmäßige Stentstruktur, die die Gefäßinnenoberfläche relativ geschlossen abgedeckt, verhindert.

Derartige Stents mit einer sogenannten geschlossenen Struktur sind ebenfalls aus dem Stand der Technik bekannt. Beispielhaft sei hier einer der bekanntesten derartiger Stents herausgegriffen, der sogenannte Wallstent. Dieser ist beispielsweise aus dem US 4,655,771 bekannt. Dieser eine geschlossene Struktur aufweisende Stent ist aus zweien gleichmäßig maschenartig gewirkten, in Längsachse des Stents spiralförmig verlaufenden Drähten gebildet.

Der Vorteil der geschlossenen Struktur derartiger Stents wird jedoch durch den Nachteil erkauft, daß diese Stents eine relative longitudinale Steifheit während des Einsetzens aufweisen. Diese Stents erlauben es daher nicht in optimaler Weise, den Stent beim Einführen in Richtung auf die zu behandelnde Stenose durch möglicherweise sehr stark gekrümmte Gefäßabschnitte der Herzkranzarterien zu führen. Um diese Nachteile einer geschlossenen Struktur zu vermeiden, wurden nunmehr Stents entwickelt, die einen sogenannten modularen Aufbau aufweisen. Bei diesen modularartig aufgebauten Stents sind einzelne, mit einer geschlossenen Struktur versehene Abschnitte, durch flexible Verbindungen miteinander verbunden. Derartige Stents sind beispielsweise aus dem US-Patent 5,104,404 bekannt.

Nachteilig bei diesen modularen bzw. segmentierten Stents ist es jedoch, daß sich die in Einführrichtung des Stents vorne liegenden Vorderkanten der einzelnen Module bzw. Segmente in der Gefäßinnenwand verhaken können. Auf diese Weise kann es zu erheblichen Komplikationen bei dem Einführen eines Stents kommen. Dies ist insbesondere insoweit besonders problematisch, da die modularen Stents - wie oben bereits ausgeführt - vor allem dann zum Einsatz gelangen, wenn erhebliche Krümmungen auf dem Weg zu der behandelnden Stelle zu überwinden sind. Denn in einer solchen Krümmung verhakt sich eine solche Vorderkante eines Segmentes besonders leicht an der in der Krümmung außenliegenden Innenoberfläche des Gefäßes, durch die der Stent hindurchgeführt wird.

Aufgabe der Erfindung ist es daher, die vorgenannten Nachteile zu vermeiden und einen Stent der eingangs genannten Art zur Verfügung zu stellen, welcher sowohl an dem Ort der zu behandelnden Stenose eine geschlossene Abdeckung der Gefäßinnenoberfläche ermöglicht, während er gleichzeitig ausreichend flexibel ist, um an diesem Ort verschoben werden zu können.

Diese Aufgabe wird bei der Erfindung mit einem Stent der eingangs genannten Art dadurch gelöst, daß die in dem Stent vorgesehenen Ausnehmungen eine im wesentlichen V-förmige Ausnehmungsfläche aufweisen.

Die Vorteile der Erfindung liegen insbesondere darin, daß durch die V-förmigen Ausnehmungen, d.h. durch die V-förmige Form der Ausnehmungsfläche der Ausnehmungen, in der rohrförmigen Struktur des Stents sowohl eine insgesamt geschlossene Struktur realisiert wird, so daß eine gute Abdeckung der Läsion erreicht wird, während gleichzeitig aufgrund der V-förmigen Ausnehmungen eine gegenüber den aus dem Stand der Technik bekannten geschlossenen Stentstrukturen erhöhte Flexibilität in longitudinaler Richtung des Stents erreicht wird.

Die Erfindung schließt die Erkenntnis ein, daß bei in longitudinaler Richtung des Stents in seinem rohrförmigen Abschnitt angeordneten, V-förmigen Ausnehmungen im Bereich der zulaufenden Spitze der Ausnehmungsfläche der V-förmigen Ausnehmungen ein in transversaler Richtung des Stents beweglicher Bereich des die Ausnehmungsfläche begrenzenden Materials entsteht, der beim Hindurchschieben des Stents durch Krümmungen beispielsweise in Herzkranzgefäßen eine ausreichende Flexibilität des Stents erlaubt, so daß diese Krümmungen leicht überwunden werden können.

Eine vorteilhafte Ausführungsform der Erfindung zeichnet sich durch erste Ausnehmungen einer ersten Größe, und durch zweite Ausnehmungen mit einer zweiten Größe aus, wobei die zweiten Ausnehmungen größer sind als die ersten Ausnehmungen. Auf diese Weise wird die vorstehend erwähnte Flexibilität der geschlossenen Struktur weiter verbessert, da sich die kleinen Ausnehmungen innerhalb der großen Ausnehmungen, insbesondere wenn sie in diese hineinverschachtelt sind, noch stärker bei Krümmung des Stents beim Hindurchschieben desselben durch gekrümmte Herzkranzgefäße transversal bewegen können.

Eine weiter bevorzugte Ausführungsform der Erfindung zeichnet sich dadurch aus, daß in longitudinaler Richtung des rohrförmigen Abschnittes des Stents erste und zweite Ausnehmungen abwechselnd aufeinander folgen. Auf diese Weise wird ein optimaler Kompromiß zwischen geschlossener Struktur und erhöhter Flexibilität des Stents in transversaler Richtung erreicht.

Eine weiter bevorzugte Ausführungsform der vorliegenden Erfindung zeichnet sich dadurch aus, daß in Umfangsrichtung des rohrförmigen Abschnittes des erfindungsgemäßen Stents die ersten und zweiten Ausnehmungen abwechselnd aufeinander folgen. Bei dieser Ausführungsform wird somit der bereits für die wechselweise Anordnung der Ausnehmungen in longitudinaler Richtung erwähnte Vorteil durch die wechselweise Anordnung der Ausnehmungen in Umfangsrichtung erreicht. Die Vorteile beider Ausführungsformen können dabei auch kombiniert werden, so daß sowohl in Umfangsrichtung als auch in longitudinaler Richtung jeweils erste und zweite Ausnehmungsgrößen alternierend aufeinander folgen.

Eine weiter vorteilhafte Weiterbildung des erfindungsgemäßen Stents zeichnet sich dadurch aus, daß die zweiten Schenkel, die das V der zweiten Ausnehmung bilden, länger sind als die ersten Schenkel, die das V der ersten Ausnehmungen bilden. Auf diese Weise läßt sich die erfindungsgemäße Struktur des Stents weiter auflockern. Durch die bei diesem Ausführungsbeispiel mögliche, alternierende Anordnung von langen und kurzen Ausnehmungen lassen sich erneut die vorstehend genannten Vorteile des erfindungsgemäßen Stents, nämlich geschlossener Struktur bei erhöhter Flexibilität, verstärken.

Bei einer weiter vorteilhaften Weiterbildung des erfindungsgemäßen Stents liegen alle in longitudinaler Richtung aufeinanderfolgenden Ausnehmungen mit ihren das spitze Ende des V bildenden Enden im wesentlichen auf einer sich ebenfalls longitudinal bezüglich des rohrförmigen Abschnittes erstreckenden Linie. Bei dieser Ausführungsform wird somit vorteilhaft ein Verhaken der einzelnen Ausnehmungsbegrenzungen, die bevorzugt aus kleinen stegartigen Elementen bestehen, miteinander verhindert, wenn sich der Stent in einer Krümmung befindet. Denn durch die auf einer Linie liegenden Enden der V-förmigen Ausnehmungen weisen diese alle den gleichen Abstand zur benachbarten Ausnehmung auf.

Eine weiter bevorzugte Ausführungsform der vorliegenden Erfindung zeichnet sich dadurch aus, daß alle in Umfangsrichtung aufeinanderfolgenden Ausnehmungen mit ihren spitzen Enden im wesentlichen auf einer sich in Umfangsrichtung des rohrförmigen Abschnittes des Stents erstreckenden Linie liegen. Auch hierdurch wird die im vorstehenden Abschnitt beschriebene vorteilhafte Struktur zur Vermeidung von Verhakungen bei gekrümmten Stent weiter perfektioniert.

Eine weitere Fortbildung der vorliegenden Erfindung zeichnet sich dadurch aus, daß die Ausnehmungen einen rechten und einen linken Schenkel aufweisen, wobei der rechte/linke Schenkel der Ausnehmungen an dem linken/rechten Schenkel der jeweiligen, im wesentlichen diagonal in Richtung des rechten/linken Schenkels der Ausnehmungen versetzt angeordneten, benachbarten Ausnehmungen endet. Auf diese Weise läßt sich vorteilhaft eine optimale Kraftübertragung zwischen den als Begrenzungen für die Ausnehmungen vorteilhaft dienenden stegartigen Elementen durch die Struktur des Stents hindurch realisieren. Dabei ist es besonders vorteilhaft, wenn der rechte/linke Schenkel der Ausnehmungen im wesentlichen in der Mitte des linken/rechten Schenkels der diagonal in Richtung des rechten/linken Schenkels der Ausnehmungen versetzt angeordneten, benachbarten Ausnehmung endet. Auf diese Weise geschieht die Kraftübertragung von einer Ausnehmung zur diagonal daneben liegenden Ausnehmung direkt auf das Zentrum des Schenkels der Ausnehmung, so daß eine optimale Krafteinleitung in die benachbarten Ausnehmungen bzw. deren stegartige Begrenzungen erreicht wird.

Eine weitere vorteilhafte Weiterbildung der vorliegenden Erfindung zeichnet sich dadurch aus, daß die Ausnehmungen von Stegen begrenzt sind, und daß die zweiten Ausnehmungen begrenzenden Stege auch die ersten Ausnehmungen begrenzen. Auf diese Weise läßt sich der rohrförmige Abschnitt gemäß der Erfindung mit maximaler Materialersparnis gestalten. Auf diese Weise wird der besondere Vorteil erreicht, daß der erfindungsgemäße Stent problemlos auch im Bereich von Gefäßverzweigungen der Herzkranzgefäße verwendet werden kann. Denn durch die sehr luftige und durchlässige Struktur kann der Stent auch zur Stützung einer Gefäßverengung im Bereich einer Gefäßverzweigung eingesetzt werden, ohne daß der Blutfluß von dem Gefäß mit dem Stent in das abzweigende Gefäß übermäßig behindert wird.

Eine weitere bevorzugte Ausführungsform der Erfindung zeichnet sich dadurch aus, daß die Ausnehmungen von Stegen begrenzt sind, wobei die Stege aus dem restlichen Material eine die Mantelfläche bildenden Rohrwandung des rohrförmigen Abschnittes gebildet sind, von welcher Rohrwandung das Material im Bereich der Ausnehmungen entfernt wurde, und wobei die die zweiten Ausnehmungen begrenzenden Stege das gesamte, nach Bildung der Ausnehmungen verbleibende Material der Rohrwandung bilden. Auch diese Ausführungsform zeichnet sich vorteilhaft dadurch aus, daß sie bei geschlossener Struktur trotzdem eine Verwendung im Bereich von Gefäßverzweigungen bei gleichzeitiger hoher Flexibilität der gesamten Struktur erlaubt.

Eine weitere Verbesserung der Flexibilität des erfindungsgemäßen Stents wird bei einer Weiterbildung der Erfindung dadurch erreicht, daß mindestens eines der Enden der Schenkel der V-förmigen Ausnehmungen abgerundet ausgebildet ist. Bevorzugt sind dabei alle Enden des V abgerundet ausgebildet. Diese runden, bevorzugt durch die stegartigen Elemente gebildeten Begrenzungen der V-förmigen Ausnehmungen verbessern weiter die Flexibilität und erhöhen auch die Zuverlässigkeit des Stents, denn sie vermeiden Spitzenbelastungen in den Enden der V-förmigen Ausnehmungen. Als besonders vorteilhaft hat sich in diesem Zusammenhang erwiesen, wenn die einzelne Spitze der V-förmigen Ausnehmung durch einen dreiviertelkreisförmigen Steg abgerundet begrenzt wird.

Eine weitere Erhöhung der zuvor ausführlich dargelegten Flexibilität des erfindungsgemäßen Stents bei gleichzeitiger geschlossener Struktur läßt sich dadurch erzielen, daß bei einer vorteilhaften Ausführungsform die ersten Ausnehmungen mit den Enden ihrer Schenkel leichte Einbuchtungen in den Schenkeln der zweiten Ausnehmungen bilden. Die dabei bevorzugt durch stegförmige Elemente gebildeten Begrenzungen der Ausnehmungen in der Mantelfläche des Stents enden somit elastisch in der diagonal benachbarten Ausnehmung. Diese Elastizität der diagonalen Begrenzungen der Ausnehmungen erhöht ebenfalls die Flexibilität des erfindungsgemäßen Stents für transversale Krümmungsbewegungen.

Weitere bevorzugte Ausführungsformen der Erfindung sind in den Unteransprüchen angegeben.

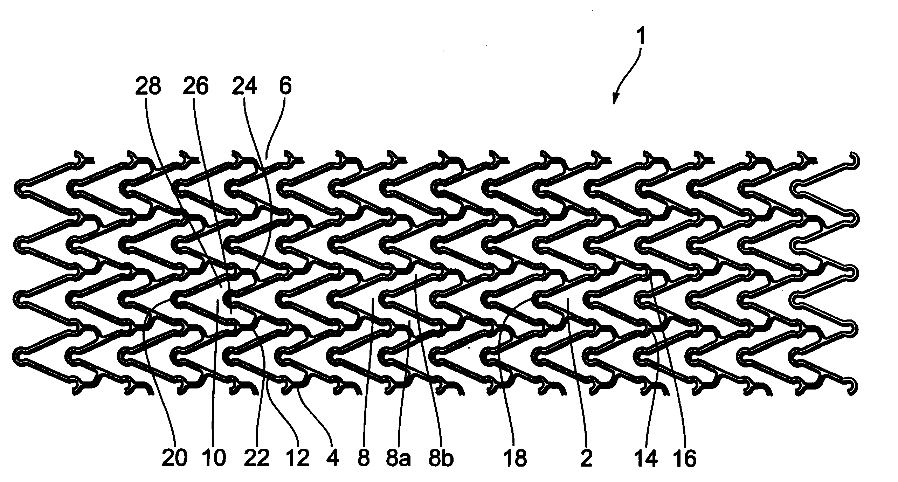

Ein Ausführungsbeispiel der vorliegenden Erfindung wird nun anhand der einzigen Figur beschrieben.

Die Figur zeigt einen erfindungsgemäßen Stent 1. Der Stent 1 ist in der Figur als Abwicklung der Mantelfläche 2 des Stents 1 dargestellt. Im funktionsfähigen Zustand des Stents 1 ist die Mantelfläche 2 mit ihrer in der Figur unten dargestellten Seite 4 und in der Figur oben dargestellten Seite 6 aneinandergefügt, so daß sich ein die Mantelfläche 2 aufweisender rohrförmiger Abschnitt ergibt.

Die Mantelfläche 2 weist erste, V-förmige Ausnehmungen 8 auf. Diese ersten V-förmigen Ausnehmungen 8 sind in diagonaler Richtung der Mantelfläche nebeneinander angeordnet. Die V-förmigen Ausnehmungen 8 weisen dabei in der Figur unten liegende, rechte Schenkel 8a auf, und in der Figur oben liegende, linke Schenkel 8b auf. Die Schenkel 8a und 8b bilden das V der V-förmigen Ausnehmungen 8. Die Ausnehmungen 8 sind auf der Mantelfläche 2 derart nebeneinander angeordnet, daß die Enden der Schenkel 8a bzw. 8b jeweils in der Mitte des benachbarten Schenkels 8b bzw. 8a der diagonal daneben liegenden Ausnehmung 8 enden. So endet der rechte Schenkel der in der Zeichnung herausgegriffenen Ausnehmung 8, der Schenkel 8a in der Mitte des diagonal rechts darunter in der Zeichnung dargestellten linken Schenkels 8b der diagonal rechts darunter benachbarten Ausnehmung 8.

Da sich die zuvor beschriebene diagonale Aneinanderreihung der V-förmigen Ausnehmungen 8 in beiden Diagonalen auf der Mantelfläche 2 zeigt, schließen die V-förmigen Ausnehmungen 8 weitere, jedoch kleinere V-förmige Ausnehmungen 10 zwischen sich ein. Eine solche kleinere V-förmige Ausnehmung 10 ist somit von vier größeren V-förmigen Ausnehmungen 8 umgeben.

Die Ausnehmungen 8 sind allseits von Stegen 12 begrenzt. Diese Stege 12 sind das verbleibende Material der Mantelfläche 2, nachdem das Material der Mantelfläche 2 an den Stellen der Ausnehmungen 8 und 10 entfernt wurde. Die Stege 12 verlaufen alle im wesentlichen parallel zu den Diagonalen der Mantelfläche 2. Darüber hinaus sind die Stege 12 dort, wo die Schenkel 8a und 8b der größeren Ausnehmungen 8 enden, d.h. an den Punkten 14 und 16 jeweils dreiviertelkreisförmig ausgebildet. Diese dreiviertelkreisförmige Ausbildung befindet sich ebenfalls am spitzen Ende 18 der großen V-förmigen Ausnehmung 8. Darüber hinaus ist auch das spitze Ende der kleinen V-förmigen Ausnehmung 10 mit einem derartig dreiviertelkreisförmig abgerundeten Steg 20 ausgestattet.

Die Enden 22 bzw. 24 der rechten Schenkel 26 bzw. linken Schenkel 28, wobei die linken Schenkel 28 in der Figur oben dargestellt sind, während die rechten Schenkel 26 in der Figur unten dargestellt sind, werden durch viertelkreisförmig ausgebildete Stege 12 gebildet. Diese Viertelkreisform der Stege 12 an den Enden 22 und 24 der Schenkel 26 und 28 bilden kleine Ausbuchtungen, die sich in die Schenkel 8b bzw. 8a der größeren V-förmigen Ausnehmungen 8 hinein erstrecken.

## Patentansprüche

1. Stent, insbesondere Koronarstent, mit einem rohrförmigen Abschnitt, der Ausnehmungen (8, 10) aufweist, dadurch gekennzeichnet, daß die Ausnehmungen (8, 10) im wesentlichen V-förmig ausgebildet sind.

2. Stent nach Anspruch 1, mit ersten Ausnehmungen (10) einer ersten Größe, mit zweiten Ausnehmungen (8) einer zweiten Größe, wobei die zweiten Ausnehmungen (8) größer sind als die ersten Ausnehmungen (10).

3. Stent nach Anspruch 2, wobei in longitudinaler Richtung des rohrförmigen Abschnittes die ersten (10) und zweiten (8) Ausnehmungen abwechselnd aufeinander folgen.

4. Stent nach einem der Ansprüche 2 oder 3, wobei in Umfangsrichtung des rohrförmigen Abschnittes die ersten (10) und zweiten (8) Ausnehmungen abwechselnd aufeinander folgen.

5. Stent nach Anspruch 4, wobei die zweiten Schenkel (8a, 8b), die das V der zweiten Ausnehmung (8) bilden, länger sind als die ersten Schenkel (26, 28), die das V der ersten Ausnehmung (10) bilden.

6. Stent nach einem der vorstehenden Ansprüche, wobei alle in longitudinaler Richtung aufeinander folgenden Ausnehmungen (8, 10) mit ihren spitzen Enden (18, 20) im wesentlichen auf einer sich longitudinal bezüglich des rohrförmigen Abschnittes erstreckenden Linie liegen.

7. Stent nach einem der vorstehenden Ansprüche, wobei alle in Umfangsrichtung aufeinander folgenden Ausnehmungen (8, 10) mit ihren spitzen Enden (18, 20) im wesentlichen auf einer sich in Umfangsrichtung des rohrförmigen Abschnittes erstreckenden Linie liegen.

8. Stent nach einem der vorstehenden Ansprüche, wobei die Ausnehmungen (8, 10) einen rechten (8a, 26) und einen linken (8b, 28) Schenkel aufweisen, und wobei der rechte (8a, 26)/linke (8b, 28) Schenkel der jeweiligen im wesentlichen diagonal in Richtung des rechten (8a, 26)/linken (8b, 28) Schenkels der Ausnehmungen (8, 10) versetzt angeordneten, benachbarten Ausnehmung (8, 10) endet.

9. Stent nach Anspruch 8, wobei der rechte (8a, 26)/linke (8b, 28) Schenkel der Ausnehmungen (8, 10) im wesentlichen in der Mitte des linken (8b, 28)/rechten (8a, 26) Schenkels der diagonal in Richtung des rechten (8a, 26)/linken (8b, 28) Schenkels der Ausnehmungen (8, 10) versetzt angeordneten, benachbarten Ausnehmung (8, 10) endet.

10. Stent nach einem der Ansprüche 2 bis 9, wobei die Ausnehmungen (8, 10) von Stegen (12) begrenzt sind, und wobei die die zweiten Ausnehmungen (8) begrenzenden Stege (12) auch die ersten Ausnehmungen (10) begrenzen.

11. Stent nach einem der Ansprüche 2 bis 9, wobei der rohrförmige Abschnitt eine Mantelfläche (2) aufweist, wobei die Ausnehmungen (8, 10) von Stegen (12) begrenzt sind, wobei die Stege (12) aus dem restlichen Material einer die Mantelfläche (2) bildenden Rohrwandung des rohrförmigen Abschnittes gebildet sind, von welcher Rohrwandung das Material im Bereich der Ausnehmungen (8, 10) entfernt wurde, und wobei die die zweiten Ausnehmungen (8) begrenzenden Stege (12) das gesamte, nach Bildung der Ausnehmungen (8, 10) verbleibende Material der Rohrwandung bilden.

12. Stent nach einem der vorstehenden Ansprüche, wobei mindestens eines der Enden (18, 20, 22, 24) der Schenkel (8a, 8b, 26, 28) der Ausnehmungen (8, 10) abgerundet ausgebildet ist.

13. Stent nach einem der vorstehenden Ansprüche, wobei die Spitze (18, 20) des V der V-förmigen Ausnehmungen (8, 10) abgerundet ausgebildet ist.

14. Stent nach einem der vorstehenden Ansprüche, wobei alle Ausnehmungen (8, 10) in der Mantelfläche (2) V-förmig ausgebildet sind.

15. Stent nach einem der Ansprüche 2 bis 14, wobei die ersten Ausnehmungen (10) mit den Enden (22, 24) ihrer Schenkel (26, 28) leichte Einbuchtungen in den Schenkeln (8b, 8a) der zweiten Ausnehmungen (8) bilden.
